# EUROPEAN PATENT APPLICATION

(11) **EP 1 978 461 A1**
(43) Date of publication of application: **08.10.2008**
(21) Application number: 07105735.0
(22) Date of filing: 05.04.2007
(51) Int. Cl.: G06F 19/00, A63B 24/00

(54) **Method and system to provide personalized advice for training**

(71) Applicant: Koninklijke Philips Electronics N.V., 5621 BA Eindhoven (NL)
(72) Inventor: The designation of the inventor has not yet been filed
(74) Representative: Schoenmaker, Maarten

(57) **Abstract**

The present invention provides a method, and a corresponding system, to provide personalized advice for training, wherein the training comprises a plurality of training sessions, the method comprising the steps of: - storing first data concerning fixed personal profile information of a user; - storing second data concerning varying personal profile information of the user; - updating the second data before each subsequent training session of the plurality of training sessions; - processing the first and second data to determine the personalized advice; and providing the personalized advice.

## Description

### FIELD OF THE INVENTION

The present invention is concerned with a method and a system to provide personalized advice for training such as exercise, fitness and/or weight control programs.

### BACKGROUND OF THE INVENTION

Good exercise programs are difficult to set up, currently needing the involvement of a professional trainer, i.e. a person. The trainer can provide advice based on diverse, personal input and output parameters. The involvement of the personal trainer is however time consuming and expensive.

EP-1 159 989-A1 provides a method and a system for generating and/or adjusting a training schedule based on personal data and optionally on preferences of a particular user. The system comprises a database containing training schedules designed by experts, such as athletes and coaches. An interpolator generates a personalized training schedule by electronically selecting a training schedule from the database in accordance with the personal data and preferences of the user. Although the existing system may often provide a suitable training schedule, the interpolator may still have to be monitored by a professional trainer to assist and instruct the interpolator in unusual cases.

### OBJECT OF THE INVENTION

The present invention aims to improve the adaptability of the system and method referred to above.

### SUMMARY OF THE INVENTION

The present invention provides a method to provide personalized advice for training, wherein the training comprises a plurality of training sessions, the method comprising the steps of: - storing first data concerning fixed personal profile information of a user; - storing second data concerning varying personal profile information of the user; - updating the second data before each subsequent training session of the plurality of training sessions; - processing the first and second data to determine the personalized advice; and - providing the personalized advice.

By updating the second data before each training session, the method provides improved adaptability to unusual circumstances and features. For instance, at a certain date and time, the training session may be at a certain location. The method of the present invention will be able to provide advice that is adapted to these specific, personal circumstances. The invention thus provides a method that has improved flexibility and can provide an improved, more personal advice than prior art methods. The method thus obviates the need for an expensive personal trainer.

In an embodiment, the first data comprise physical or mental properties, such as medical history, health, age, gender, and/or education level.

In a further embodiment, the second data comprise habits and preferences of the user, in particular eating pattern, drinking pattern, music preferences, motivation, coaching style, sports, activities, fitness level, perseverance, endurance, condition, and/or way of communication. The second data may also comprise preferences of a training session of the plurality of training sessions, in particular time, date, location, climate, food, temperature, weather conditions, and/or air quality at the location of the respective training session.

In an embodiment, the step of processing the first and second data to determine the personalized advice comprises the steps of:
- contacting a database including reference user profiles and corresponding training advice;
- pattern matching the first data and the second data to the reference user profiles to provide a best matching reference user profile; and
- providing the best matching reference user profile as the personalized advice.

The above embodiment may use a pattern matching algorithm to provide the advice. The advice is up to date due to updating of the second data before each subsequent training session. Also the database may be updated regularly to provide the best possible and most up to date advice. Pattern matching may be executed at a personal device, such as a PDA or other handheld computer, or at a remote location. At the remote location, a central server system, which may be updated by a service provider, can execute the pattern-matching step. The latter provides the possibility of relatively powerful, centrally located equipment, and simplifies maintenance.

In another embodiment, the step of pattern matching the first and/or second data to the reference user profiles to provide a best matching reference user profile includes the step of - pattern matching a mismatching part of the best matching reference user profile to the reference user profiles, to adjust the mismatching part of the best matching reference user profile. Thus the pattern-matching step provides fine-tuning of the advice by matching mismatching parts of the user profile, and amending the corresponding advice accordingly.

In another embodiment, the step of processing the first and second data to determine the personalized advice comprises the steps of: - contacting a rules engine comprising rules that are based on the first data; - supplying the second data to the rules engine to provide the personalized advice. This method provides a relatively simple and robust way to provide the personalized advice. Updating the second data assures that the advice is up to date, and corresponds to for instance local conditions at the site of the training session.

Preferably, the step of updating the second data before each subsequent training session of the plurality of training sessions comprises the step of: - contacting the internet, wherein the database and/or the rules engine comprise at least part of the internet. As the internet can be regarded as the largest database in the world, using part of the internet as database to update the second data increases the accuracy of the advice provided by the method of the present invention.

According to another aspect, the present invention provides a system to provide personalized advice for training, wherein the training comprises a plurality of training sessions, the system comprising:
- a first memory comprising first data concerning fixed personal profile information of a user;
- a second memory comprising second data concerning varying personal profile information of the user;
- input means that are adapted to update the second data before each subsequent training session of the plurality of training sessions;
- processing means that are adapted to process the first and second data to determine the personalized advice; and
- output means that are connected to the processing means to output the personalized advice.

As the input means are adapted to update the second data before each training session, the system provides improved adaptability to unusual circumstances and features. The system of the present invention will be able to provide advice that is adapted to these specific, unusual circumstances. The invention thus provides a system having improved flexibility, which can provide an improved, more personal advice than prior art methods. The system thus obviates the need for an expensive personal trainer.

In an embodiment, the processing means include:
- a database including reference user profiles and a corresponding training advice, wherein each reference user profile is coupled to a respective training advice;
- pattern matching means that are adapted to pattern match the first data and the second data and the reference user profiles to provide a best matching reference user profile;
   wherein the processing means are adapted to provide the training advice that is coupled to the best matching reference user profile as the personalized advice.

The system of the above embodiment may include a pattern matching algorithm to provide the advice. The advice is up to date due to updating of the second data before each subsequent training session. Also the database may be updated regularly to provide the best possible and most up to date advice. The system may include a personal device, such as a PDA or other handheld computer, to execute the pattern matching. Alternatively, the system may include a device at a remote location to execute the pattern-matching. The device at the remote location may include a central server system, which may be updated by a service provider. The latter provides the advantage of relatively powerful, centrally located equipment, including simplified and cost effective maintenance.

In another embodiment, the processing means are adapted to pattern match a mismatching part of the best matching reference user profile with the reference user profiles, to adjust the mismatching part of the best matching reference user profile. Thus the processing means allow fine-tuning of the advice to user specific information. Thus, the system of the present invention provides an improved adaptability to unusual cases.

In a further embodiment, the processing means include: - a rules engine comprising rules that are based on the first data; wherein the rules engine is adapted to determine the personalized advice using the rules and the second data. This provides a relatively robust system.

### BRIEF DESCRIPTION OF THE DRAWINGS

Further features and advantages of the present invention will be apparent from the following description and the enclosed drawings, wherein:
Fig. 1 shows a schematic representation of an embodiment of a system according to the present invention; and
Fig. 2 shows a schematic representation of another embodiment of a system according to the present invention.

### DETAILED DESCRIPTION OF EXAMPLES

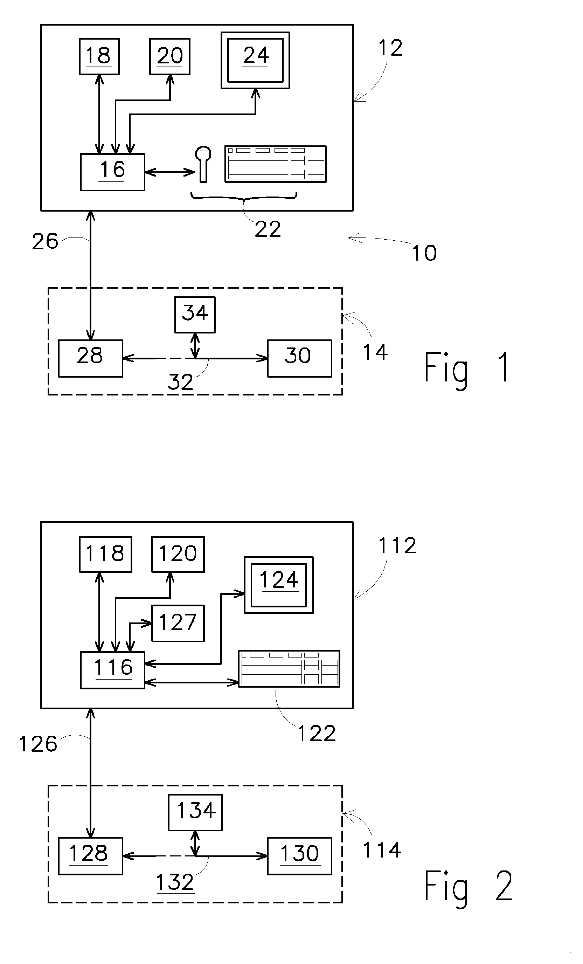

Fig. 1 shows an embodiment of the system 10 to provide personalized advice for training. In the embodiment, the system 10 comprises a portable device 12, which is coupled to a remote device 14. The portable device 12 comprises a processor 16, a first memory 18, a second memory 20. Also, the portable device 12 comprises input means 22 and output means 24.

The device 12 is coupled to the other device 14 via data connection 26. The device 14, which may be located remote from the device 12, comprises a database 28. Optionally, the database 28 is coupled to an update device 30 via data connection 32.

The input means 22 for instance comprise a keyboard, a microphone, and/or a computer mouse. The output means 24 for instance comprise a monitor, one or more lights, and/or a speaker.

The first memory 18 comprises first data, which are part of a detailed user profile of the user of the device 12. The first data for instance comprise fitness level, medical history, age, gender, and/or education level.

The second memory 20 comprises second data, which are another part of the detailed user profile. The second data comprise for instance eating pattern, drinking pattern, music preferences, motivation, coaching style, sports, activities, and/or way of communication. In an embodiment, the second data comprise time, date, training location, local climate, local food, local temperature, local weather conditions, and/or local air quality.

In a second embodiment, system 110 comprises a device 112, which is coupled to device 114. The device 112 may be portable and comprises a processor 116, a first memory 118 and a second memory 120. Coupled to the processor 116 are input means 122 and output means 124. The device 112 is coupled to the device 114 via data connection 126. The device 112 comprises a first part of rules engine 127. All elements of the portable device 112 are coupled to each other via or controlled by the processor 116.

A second part of the rules engine 128 is comprised in the device 114. The device 114 further comprises update device 130 which is coupled to the rules engine 128 via data connection 132. Optionally, the device 114 comprises second processor 134 which is coupled to the data connection 132.

The operation of the system 10 shown in Fig. 1 is based on the first memory 18 and the second memory 20, in connection with the database 28. The database 28 comprises existing, reference user profiles. The reference user profiles preferably comprise information concerning the same features as the detailed user profile, stored in the first and second memory, as well as corresponding training schedules and/or training advice. The training advice of the reference profiles is preferably formulated by professional trainers, and thus comprises years of expertise.

Before starting training, the user of device 12 inputs specific information about the training, using for instance input means 22. The specific information preferably concerns the second data. For instance, the input may comprise whether the training will be inside or outside, the location of the training, the duration of the training, the time and/or amount of breakfast that morning, and/or his condition in general.

After inputting the information, the processor 16 updates the user profile stored in the first memory 18 and second memory 20 accordingly. The processor 16 sends the updated user profile to database 28, and subsequently a pattern matching algorithm is executed. The pattern matching may be executed by the processor 16, or in an embodiment by another processor 34, which is comprised in device 14.

The training advice accompanying the reference user profile in database 28 that best matches the updated user profile may be copied as training advice and send to device 12.

Fine-tuning of the training advice accompanying the reference user profile in database 28 that best matches the updated user profile is possible. In an embodiment, each feature or aspect that mismatches significantly with the reference user profile is taken apart. The mismatching feature is, separate from the original detailed user profile, matched with the reference user profiles in the database 28. The corresponding part of the training advice accompanying the best matching reference profile replaces part of the above mentioned training advice.

As an example of fine-tuning, imagine that a user has asthma. His detailed profile matches a reference profile of a reference person without asthma. The training advice of the latter reference person can be used as training advice, except in conditions that would influence the asthma. Examples of said conditions are (outdoor) pollution level, and/or dust. Thus, if the updated user profile indicates that the user will train at a certain time, date, and/or location having a pollution level that exceeds a predetermined maximum pollution level for asthma, the training advice will for instance advice to train with the same effort at another location, e.g. indoors.

In the second embodiment of Fig. 2, the user of device 112 inputs specific information about the training, using for instance input means 122, before starting the training. The specific information preferably concerns the second data. For instance, the input may comprise whether the training will be inside or outside, the location of the training, the duration of the training, the time and/or amount of breakfast that morning, and/or his condition in general.

After inputting the information, the processor 116 updates the user profile stored in the first memory 118 and second memory 120 accordingly. The processor 116 sends the updated user profile to the first part of the rules engine 127. Subsequently, parts of the user profile for which no rules are available in the first part 127, and/or parts of the user profile that have been altered since a previous training, are send to the second part of the rules engine 128. The second part 128 obtains, optionally controlled by the second processor 134, updated rules from the update device 130 via the data connection 132, together with corresponding information. The second part of the rules engine sends the updated rules to the first part of the rules engine 127. The first part 127, together with the processor 116, determines the personalized training advice using the updated set of rules.

As an example, again imagine the user of device 112 having asthma. One of the rules could read "as long as the outdoor pollution level exceeds a predetermined threshold value, the user is dissuaded from training outdoors". The input in this case would include the location of the forthcoming training.

In the latter case, the processor 116 could propose another training location indoors. Again, the rules engine 127 will check the forthcoming training, including the newly proposed training location. If the new training satisfies all rules in the first part of the rules engine 127, the processor outputs the training advice including the new training location via output means 124.

The rules engine 127 may also include generic rules, for instance to determine a suitable time of training. For example, after a light supper the user can be advised to start training half an hour after the meal. In case of a heavy or alcohol-accompanied dinner evening training will be cancelled and the next training adapted accordingly.

In a practical embodiment, the device 14, 114 may be incorporated in the Internet. Alternatively, the update device 30, 130 may be incorporated in the Internet.

The device 12, 112 may be a suitable portable device, such as a PDA, or a smart phone. The data connection 26, 126 between the phone and the remote device 14, 114 may include a (wireless) telecom network such as a GSM network.

A training session as described above may include one or more of:
- an exercise routine,
- a fitness training, comprising one or more of weight lifting, running, cycling, aerobics, and/or rowing;
- preventive or personal healthcare,
- a diet, comprising eating habits and patterns, daily calorie intake, and/or food composition concerning the percentage of fat, protein and carbohydrates of the food that the user consumes per day; and/or
- a weight loss program.

After or during a work out or training session, people tend to have a subjective perception of the effort spent during the training session. Subjective measures may influence the mood of the person, and thus influence the motivation of the person.

The training session or work out generally has a positive effect on the mood, health and appearance of the person. The short-term effect of the training session may be indicated by the effort of the person during the training session. The effort may be indicated using for instance number of calories spent, number of repetitions of a certain exercise, and/or distance of a certain run or bike ride. However, the medium and long-term effects of the training are hard to access. To determine medium and long-term effects, computing means may be needed, and the user needs to access the computing means to obtain the calculated results. The user needs to start up the computing means, which generally means the start up of an electronic device.

In an embodiment, the present invention aims to render the effort or result of training, comprising a sequence of training sessions, more accessible.

The calculated effort of the sequence of training sessions is therefore indicated in the environment of the user. Indicating the effort and/or result motivates the user in continuing the training. Good results may impress others, thus improving for instance the status of the user or making him or her feel good. Whereas bad results may motivate the user to increase his or her efforts, and thus to improve the results. Such challenges will positively influence the motivation of the user to perform during training.

The environment herein comprises for instance the home of the user, the gym, a personal device that is portable and might be carried by the user at certain times. Examples of the personal device include a watch, a portable phone.

The system of the present invention includes display means to display the results of the training. Display of the results herein comprises substantially continuously display. Display of the results obviates the need to use a screen or monitor. The display means therefore comprise for instance clothing, furniture, and bedclothes. The display means include suitable, for instance sewn-in electronic displays.

The electronic displays may display the basic results continuously. For instance, if the results are good on average, the display means indicate a green color, whereas they indicate a red color if the results are below a predetermined threshold value. Alternatives for the examples red and green include: a predetermined pattern, color versus black and white, and/or light intensity.

In another embodiment, the system also comprises sound equipment. If the results are above the threshold value, the system produces sounds that are generally regarded as pleasant. On the other hand, the system will produce sounds that are generally regarded as unpleasant, if the results are on average below the threshold value or values.

The present invention is not limited to the above-described embodiments, which may either alone or in combination be amended within the scope of the appended claims.

## Claims

1. A method to provide personalized advice for training, wherein the training comprises a plurality of training sessions, the method comprising the steps of:
- storing first data concerning fixed personal profile information of a user;
- storing second data concerning varying personal profile information of the user;
- updating the second data before each subsequent training session of the plurality of training sessions;
- processing the first and second data to determine the personalized advice; and
- providing the personalized advice.

2. The method of claim 1, wherein the first data comprise physical or mental properties, such as medical history, health, age, gender, and/or education level.

3. The method of claim 1 or 2, wherein the second data comprise habits and preferences of the user, in particular eating pattern, drinking pattern, music preferences, motivation, coaching style, sports, activities, fitness level, perseverance, endurance, condition, and/or way of communication.

4. The method of claim 1, 2 or 3, wherein the second data comprise preferences of a training session of the plurality of training sessions, in particular time, date, location, climate, food, temperature, weather conditions, and/or air quality at the location of the respective training session.

5. The method of any of claims 1-4, wherein the step of processing the first and second data to determine the personalized advice comprises the steps of:
- contacting a database including reference user profiles and corresponding training advice;
- pattern matching the first data and the second data to the reference user profiles to provide a best matching reference user profile; and
- providing the best matching reference user profile as the personalized advice.

6. The method of claim 5, wherein the step of pattern matching the first and/or second data to the reference user profiles to provide a best matching reference user profile includes the step of:
- pattern matching a mismatching part of the best matching reference user profile to the reference user profiles, to adjust the mismatching part of the best matching reference user profile.

7. The method of any of claims 1-4, wherein the step of processing the first and second data to determine the personalized advice comprises the steps of:
- contacting a rules engine comprising rules that are based on the first data;
- supplying the second data to the rules engine to provide the personalized advice.

8. The method of claim 5 or 7, wherein the step of updating the second data before each subsequent training session of the plurality of training sessions comprises the step of:
- contacting the internet, wherein the database and/or the rules engine comprise at least part of the internet.

9. A system to provide personalized advice for training, wherein the training comprises a plurality of training sessions, the system comprising:
- a first memory comprising first data concerning fixed personal profile information of a user;
- a second memory comprising second data concerning varying personal profile information of the user;
- input means that are adapted to update the second data before each subsequent training session of the plurality of training sessions;
- processing means that are adapted to process the first and second data to determine the personalized advice; and
- output means that are connected to the processing means to output the personalized advice.

10. The system of claim 9, wherein the first data comprise physical or mental properties, such as medical history, health, age, gender, and/or education level.

11. The system of claim 9 or 10, wherein the second data comprise habits and preferences of the user, in particular eating pattern, drinking pattern, music preferences, motivation, coaching style, sports, activities, fitness level, perseverance, endurance, condition, and/or way of communication.

12. The system of claim 9, 10 or 11, wherein the second data comprise preferences of a training session of the plurality of training sessions, in particular time, date, location, climate, food, temperature, weather conditions, and/or air quality at the location of the respective training session.

13. The system of claim 9, wherein the processing means include:
- a database including reference user profiles and a corresponding training advice, wherein each reference user profile is coupled to a respective training advice;
- pattern matching means that are adapted to pattern match the first data and the second data and the reference user profiles to provide a best matching reference user profile;
wherein the processing means are adapted to provide the training advice that is coupled to the best matching reference user profile as the personalized advice.

14. The system of claim 13, wherein the processing means are adapted to pattern match a mismatching part of the best matching reference user profile with the reference user profiles, to adjust the mismatching part of the best matching reference user profile.

15. The system of any of claims 9-12, wherein the processing means include:
- a rules engine comprising rules that are based on the first data;
wherein the rules engine is adapted to determine the personalized advice using the rules and the second data.

16. The system of claim 13 or 15, wherein the database and/or the rules engine comprise at least part of the internet.
